# EUROPÄISCHE PATENTSCHRIFT

(11) **EP 0 608 713 B1**
(45) Veröffentlichungstag und Bekanntmachung des Hinweises auf die Patenterteilung: **27.12.1996**
(21) Anmeldenummer: 94100422.8
(22) Anmeldetag: 13.01.1994
(51) Int. Cl.: C07C 255/50

(54) **2,3-Difluor-6-nitrobenzonitril und 2-Chlor-5,6-difluorbenzonitril, Verfahren zu deren Herstellung und ihre Verwendung zur Herstellung von 2,3,6-Trifluorbenzoesäure**
2,3-Difluoro-6-introbenzonitrile and 2-chloro-5,6-difluorobenzonitrile, process for their preparation and their use in the preparation of 2,3,6-trifluorobenzoic acid
2,3-Difluoro-6-nitrobenzonitrile et 2-chloro-5,6-difluorobenzonitrile, procédé pour leur préparation et leur utilisation pour la préparation de l'acide 2,3,6-trifluorbenzoique

(30) Priorität: 23.01.1993 DE 4301756
(43) Veröffentlichungstag der Anmeldung: 03.08.1994
(73) Patentinhaber: HOECHST AKTIENGESELLSCHAFT, 65929 Frankfurt am Main (DE)
(72) Erfinder: Pfirmann, Ralf, Dr., D-64347 Griesheim (DE); Papenfuhs, Theodor, Dr., D-60433 Frankfurt am Main (DE)

(56) Entgegenhaltungen:
- EP-A- 0 180 057
- EP-A- 0 334 188
- WO-A-91/06530
- PATENT ABSTRACTS OF JAPAN vol. 15, no. 263 (C-847)4. Juli 1991 & JP-A-03 090 057 (ISHIHARA SANGYO KAISHA LTD.)
- PATENT ABSTRACTS OF JAPAN vol. 15, no. 263 (C-847)4. Juli 1991 & JP-A-03 090 057

## Beschreibung

Die vorliegende Erfindung betrifft 2,3-Difluor-6-nitrobenzonitril und 2-Chlor-5,6-difluorbenzonitril (2,3-Difluor-6-chlorbenzonitril), ein Verfahren zu ihrer Herstellung und ihre Verwendung zur Herstellung von 2,3,6-Trifluorbenzoesäure.

2,3,6-Trifluorbenzoesäure stellt ein wichtiges Zwischenprodukt zur Synthese von Pflanzenschutzmitteln und Pharmaka dar. Die Herstellung von Pestiziden der Pyrethroidreihe mit vorteilhaften Eigenschaften ist in EP 498 724 beschrieben, die Umsetzung zu Chinoloncarbonsäureantiinfektiva zum Beispiel aus US 4 874 764 bekannt.

Bis jetzt sind nur unökonomische oder technisch nicht gangbare Synthesewege zur Herstellung der 2,3,6-Trifluorbenzoesäure bekannt, so zum Beispiel über die Lithiierung (Halogen-Metall-Austausch) von 2,4,5-Trifluorbrombenzol in Ethern (J. Org. Chem. 55 (2), 773-775), die die Zielverbindung in 50 % Ausbeute neben einem entsprechendem Anteil an 2,4,5-lsomeren liefert, das zudem sehr schwer abzutrennen ist. Zuerst beschrieben wurde die Verbindung von Holland et al. (J. Org. Chem. 29 (1964), 3045), die die Entstehung der Verbindung in 69 % Ausbeute bei mechanistischen Untersuchungen der Umsetzung von selbst schwer zugänglichem 2,3,5,6-Tetrafluor-4-trifluormethyl-phenylhydrazin mit starken Basen beobachteten. Durch Umsetzung von 2,3,4-Trichlornitrobenzol über Chlor/Fluor-Austausch, Fluor-Cyanid-Austausch, anschließende denitrierende Chlorierung und erneutem Chlor/Fluor-Austausch läßt sich ebenfalls 2,3,6-Trifluorbenzonitril (JP 03090057) herstellen, das danach nach literaturbekannten Verfahren zur 2,3,6-Trifluorbenzoesäure hydrolysiert werden kann. Dieses Verfahren hat den besonderen Nachteil, daß die Wahrscheinlichkeit zur Bildung von polychlorierten Dibenzodioxinen beim ersten Chlor/Fluor-Austausch sehr hoch ist. Zudem ist es notwendig, zwei Chlor-Fluor-Austausch-(Halex)-Umsetzungsschritte bis zum Produkt durchzuführen. Des weiteren ist die erforderliche Chlor-Cyanid-Austausch-Reaktion wegen der geringen Aktivität des Chlor-Atoms ungünstig.

Es bestand somit ein Bedarf nach einem neuen Verfahren bzw. neuartigen Zwischenprodukten zur Herstellung der 2,3,6-Trifluorbenzoesäure, die die beschriebenen Nachteile nicht aufweisen.

Die Erfindung betrifft 2,3-Difluorbenzonitrile der Formel I 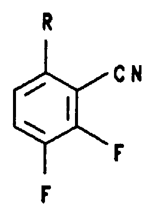 wobei R für NO₂ oder Cl steht. Sie umfaßt ferner ein Verfahren zu ihrer Herstellung und die Verwendung zur Herstellung von 2,3,6-Trifluorbenzoesäure.

Das Verfahren ist dadurch gekennzeichnet, daß man 2,3,4-Trifluornitrobenzol mit einem Cyanid in Gegenwart eines Lösungsmittels zu 2,3-Difluor-6-nitrobenzonitril umsetzt und das 2,3-Difluor-6-nitrobenzonitril gegebenenfalls mit einem Chlorierungsmittel bei erhöhter Temperatur gegebenenfalls in Anwesenheit eines fluoridfangenden und/oder wasserentziehenden Mittels zu 2-Chlor-5,6-difluorbenzonitril (2,3-Difluor-6-chlorbenzonitril) umsetzt.

Das erfindungsgemäße Verfahren verwendet 2,3,4-Trifluornitrobenzol als Ausgangsmaterial, zu dessen Herstellung mehrere ökonomisch vorteilhafte und technisch gangbare Verfahren bestehen (siehe u. a. JP 61044831, JP 63203636) und das bereits in großen Mengen ebenfalls zur Synthese antibakterieller Mittel der Chinoloncarbonsäure-Reihe (Ofloxacin, Lomefloxacin, Fleroxacin u. v. m.) technisch hergestellt wird.

Es lassen sich verschiedene Cyanide mit Erfolg einsetzen. Es können Alkali- oder Erdalkalimetallcyanide oder Cyanide von Nebengruppenelementen verwendet werden, bevorzugt Natrium-, Kalium- oder Kupfercyanid. Diese Cyanide werden in Mengen von etwa 1.0 Mol bis etwa 5 Mol, bevorzugt etwa 1.1 Mol bis etwa 2 Mol, pro 1 Mol umzusetzendes 2,3,4-Trifluornitrobenzol eingesetzt.

Bei der Umsetzung mit Cyaniden arbeitet man in dipolar aprotischen Lösungsmitteln wie Aceton, Tetrahydrofuran (THF), Acetonitril, 1,2-Dimethoxyethan (DME), Sulfolan (Tetramethylensulfon), Tetramethylensulfoxid (TMSO), N,N-Diethylacetamid, N,N-Dimethylacetamid (DMAc), N,N-Dimethylformamid (DMF), N-Methylpyrrolidon (NMP), Dimethylsulfoxid (DMSO), Dimethylsulfon, Diphenylsulfoxid, Diphenylsulfon, Tetramethylharnstoff, Tetra-n-butylharnstoff, 1,3-Dimethylimidazolidin-2-on (DMI) oder Mischungen derselben. Alternativ sind aber ebenso protische Lösungsmittel wie zum Beispiel tertiäre Alkohole, bevorzugt tert. -Butanol, oder Wasser verwendbar. Ebenso ist es möglich, in einem Überschuß von 2,3,4-Trifluornitrobenzol als Lösungsmittel zu arbeiten.

Hierbei führt man die Umsetzung bei Temperaturen von 20° bis 120° C, bevorzugt zwischen 40° und 80°C durch.

2,3-Difluor-6-nitrobenzonitril kann zum Beispiel durch Abdestillieren des Hauptteils des verwendeten Lösungsmittels und Ausfällen mit Wasser als Rohprodukt isoliert werden. Die Reinigung erfolgt zweckmäßigerweise durch Umkristallisation unter Zusatz von Aktivkohle, kann aber auch chromatographisch erfolgen.

Um 2-Chlor-5,6-difluorbenzonitril herzustellen, setzt man 2,3- Difluor-6-nitrobenzonitril mit einem Chlorierungsmittel bei erhöhter Temperatur gegebenenfalls in Anwesenheit eines fluoridfangenden und/oder wasserentziehenden Mittels um. Die denitrierende Chlorierung ist in der Literatur bekannt (EP 163230 A3, EP 355719 A1, EP 180057, EP 150587) und wird schon seit langer Zeit technisch durchgeführt. Sie sind jedoch nur an Substraten mit schwer oxidierbaren Substituenten beschrieben. Bei diesem Umsetzungstyp fällt in der Regel durch oxidativen Abbau eines Teils des Ausgangsmaterials ein stark korrosives Gemisch aus Wasser, Halogenwasserstoffen und nitrosen Gasen an.
Demzufolge war zu befürchten, daß das relativ komplex substituierte Ausgangsmaterial (2,3-Difluor-6-nitrobenzonitril) unter den Reaktionsbedingungen leichter als bekannte Substrate zersetzt würde.
Im Hinblick darauf war es überraschend, daß sich das erfindungsgemäß hergestellte neue 2,3-Difluor-6-nitrobenzonitril mit hoher Selektivität unter Ersatz eines Chloratoms umsetzen läßt, ohne daß Zersetzungsreaktionen oder erhebliche Cyanwasserstoff-Abspaltung auftreten.

Als Chlorierungsmittel lassen sich die üblichen Agentien wie Chlor, Phosphortrichlorid oder Phosphorpentachlorid, insbesondere Chlor verwenden.

Der Ersatz der Nitrogruppen durch Chlor tritt bei Temperaturen zwischen 110° und 200°C ein. Besonders einfach gestaltet sich das Verfahren, wenn man bei einer Sumpftemperatur von 175° bis 190° C arbeitet, da hierbei bereits eine genügend hohe Umsetzungsgeschwindigkeit erreicht wird und 2-Chlor-5,6-difluorbenzonitril gegebenenfalls gleichmäßig als Rohprodukt aus der Reaktionsmischung abdestilliert werden kann. Pro Mol 2,3-Difluor-6-nitrobenzonitril werden 0,5 bis 20 Mol, bevorzugt 0,7 bis 3 Mol Chlorierungsmittel verwendet. Die bevorzugt eingesetzten Chlorströme betragen zwischen etwa 10 bis etwa 400 ml/g·h, bevorzugt zwischen 20 und 200 ml/g·h. Diese Stufe kann bevorzugt diskontinuierlich unter Abdestillieren des Endproduktes erfolgen. Hierbei kann es sich auch durchaus als sinnvoll erweisen, Chlor im Unterschuß, bevorzugt im 20 bis 30prozentigen molaren Unterschuß einzusetzen und danach die leichtsiedenden Anteile, die das Produkt enthalten, abzudestillieren. In diesem speziellen Fall hat diese Arbeitsweise mit Unterschuß an Chlor wegen des relativ hohen Erstarrungspunktes des Ausgangsmaterials und der Gefahr zur exothermen Zersetzung Nachteile. Es erweist sich als zweckmäßig, dem Chlorierungssumpf zur Verhinderung der Fluorwasserstoffkorrosion, die bei solchen Umsetzungen in erheblichem Maße auftreten kann, wasserentziehende und/oder fluoridabfangende Mittel zuzusetzen, wobei Calciumsalze wie Calciumchlorid, Calciumsulfat und Calciumhydroxid sowie Siliciumdioxid bevorzugt sind. Als wasserentziehende Mittel eignen sich Phosphorpentoxid und Phosphorpentachlorid. Diese Zusätze können gegebenenfalls bis 10 Gewichtsprozent, bezogen auf die Reaktanden bei der Chlorierung, ausmachen. In den meisten Fällen genügt es, 2 bis 5 Gew.-% zu verwenden.

Ein weiterer Vorteil des erfindungsgemäßen Verfahrens besteht darin, daß das Wertprodukt bereits in einer solchen Reinheit anfällt, daß es üblicherweise unmittelbar weiterverarbeitet werden kann.

2-Chlor-5,6-difluorbenzonitril (2,3-Difluor-6-chlorbenzonitril) läßt sich vorteilhaft zur Herstellung von 2,3,6-Trifluor-benzoesäure verwenden.

2-Chlor-5,6-difluorbenzonitril wird zunächst zu 2,3,6-Trifluorbenzonitril durch Chlor-Fluor-Austausch (Halex)-Reaktion umgesetzt. Man arbeitet dabei gegebenenfalls in einem Lösungsmittel oder in einer Schmelze des Eduktes, indem man mit Alkalimetallfluoriden bei erhöhten Temperaturen, gegebenenfalls in Anwesenheit von Phasentransferkatalysatoren, umsetzt.

Man verwendet als Alkalimetallfluoride zum Beispiel Kalium-, Rubidium- oder Cäsiumfluorid oder Mischungen aus diesen in Mengen von 0.8 bis 3 Mol Fluorid pro Mol 2-Chlor-5,6-difluorbenzonitril, bevorzugt 0.9 bis 1.5 Mol, besonders bevorzugt 0.95 bis 1.2 Mol. Mischungen aus Kalium- und Cäsiumfluorid und reines Kaliumfluorid sind bevorzugt. Bei der Umsetzung des erfindungsgemäß neuen Zwischenproduktes wird die Verwendung von sprühgetrocknetem Fluoridsalz toleriert, diese ist zum Erhalt guter Ergebnisse aber nicht erforderlich.

Man arbeitet bei der Chlor-Fluor-Austausch-Stufe üblicherweise in dipolar aprotischen Lösungsmitteln wie Sulfolan (Tetramethylensulfon), Tetramethylensulfoxid (TMSO), N,N-Diethylacetamid, N,N-Dimethylacetamid (DMAc), N,N-Dimethylformamid (DMF), N-Methylpyrrolidon (NMP), Dimethylsulfoxid (DMSO), Dimethylsulfon, Diphenylsulfoxid, Diphenylsulfon, Tetramethylharnstoff, Tetra-n-butylharnstoff, 1,3-Dimethylimidazolidin-2-on (DMI) oder Mischungen derselben.

Als Phasentransferkatalysatoren kommen quartäre Ammonium- oder Phosphoniumverbindungen wie Tetraalkyl-(C₁-C₁₈)-ammoniumchloride, -bromide oder -fluoride, Tetraalkyl-(C₁-C₁₈)-phosphoniumchloride oder bromide, Tetraphenylphosphoniumchlorid oder- bromid, ((Phenyl)ₘ(alkyl(C₁-C₁₈)ₙ)-phosphoniumchloride oder -bromide, wobei m = 1 bis 3, n = 3 bis 1 und m + n = 4 ist, in Frage. Bevorzugt sind hierbei Phosphoniumsalze, besonders bevorzugt Tetraalkyl-(C₁-C₁₈)-phosphoniumbromide. Diese Stoffe werden in Mengen von 0.01 bis 50 Mol-%, bezogen auf 2-Chlor-5,6-difluorbenzonitril eingesetzt, bevorzugt zwischen 0.5 bis 10 Mol-%, besonders bevorzugt zwischen 1 und 5 Mol-%. Ebenfalls können Oligo- oder Polyethylenglykoldimethylether als Phasentransferkatalysatoren verwendet werden. Die Zahl der Glykoleinheiten in diesen Verbindungen kann von n = 4 (Tetraethylenglykoldimethylether) bis n = 150 betragen, bevorzugt werden jedoch Ether eingesetzt, deren Polymerisationsgrad zwischen n = 4 und n = 25 beträgt. Die optimale Einsatzmenge dieser Glykolether liegt zwischen 0.5 Massen-% und 200 Massen-%, bezogen auf die Masse des eingesetzten Reaktionssalzes, bevorzugt zwischen 5 und 100 Massen-%, besonders bevorzugt zwischen 10 und 50 Massen-%. Der besondere Vorteil bei der Verwendung dieser Verbindungen liegt darin, daß meist der Einsatzmenge entsprechend weniger Lösungsmittel verwendet werden kann, da die Glykolether bei der Reaktionstemperatur stets flüssig sind. Es lassen sich auch Mischungen der vorstehend genannten Phasentransferkatalysatoren in beliebiger Zusammensetzung verwenden. Eine derartige Arbeitsweise kann im Einzelfall vorteilhaft sein.

Man arbeitet bei Temperaturen zwischen 20° und 250° C, bevorzugt zwischen 120° und 200°C, besonders bevorzugt zwischen 140° und 180°C.

Das bei dieser nachfolgenden Stufe erhaltene Produktgemisch wird im allgemeinen durch Filtration des Reaktionssalzes und, besonders bei Durchführung im technischen Maßstab, anschließendes Abdestillieren der leichtflüchtigen Bestandteile und Fraktionierung erhalten. Es ist auch eine direkte Fraktionierung aus dem Filtrat möglich. Ebenfalls möglich ist, das Rohgemisch mit Wasser zu versetzen und die leichtere Oberphase, die das Produkt enthält, abzutrennen. Durch Extraktion des Wassers kann eine vollständige Abtrennung des Produktes aus der Mutterlauge erhalten werden. Danach kann durch chromatographische oder destillative Trennung eine Reinigung erfolgen.

Die Ausbeuten betragen in der Regel 75 bis 90 %, je nach Wahl des Katalysators, der Reaktionstemperatur und der Konzentration im Lösungsmittel.

Die Hydrolyse des 2,3,6-Trifluorbenzonitrils ist literaturbekannt und verläuft vollständig analog zur Umsetzung ähnlicher fluorierter Benzoesäuren (z. B. EP 433124, EP 431 373). Im allgemeinen hydrolysiert man in 70 bis 90prozentiger Schwefelsäure und erhält dabei Ausbeuten von über 90 % d. Th. (H. Henecka in Houben-Weyl-Müller, loc. cit.; s. a. Beispiel 4).

Alle Verfahrensschritte können bei Atmosphärendruck, Unter- oder Überdruck durchgeführt werden, wobei in der Stufe der denitrierenden Chlorierung die Arbeitsweise bei leichtem Überdruck bevorzugt ist. Bevorzugt in der Stufe der Halex-Reaktion ist die Arbeitsweise bei leichtem Überdruck in einem geschlossenen Gefäß, um Verlust des leichtflüchtigen Produktes zu vermeiden, da dieses bei den Reaktionstemperaturen bereits einen erheblichen Dampfdruck besitzt. Man kann diesen Effekt dazu ausnutzen, das Produkt kontinuierlich während der Reaktion abzudestillieren, wodurch aber eine aufwendigere Apparatur und Steuerungstechnik (gleichmäßiger Rücklauf) benötigt wird. Eine nachfolgende Feinfraktionierung wird aber dennoch im allgemeinen erforderlich. Bei optimaler Einstellung der Reaktionsbedingungen wie Katalysator, Konzentration, Temperatur und Salzmenge erweist sich dieser zusätzliche Aufwand aber nicht als erforderlich, um hohe Ausbeuten und Raumleistungen zu erhalten.

Die folgenden Beispiele erläutern das Verfahren.

### Beispiel 1

In 400 g tert.-Butanol werden 32.5 g (0.5 Mol) Kaliumcyanid vorgelegt und bei 70°C 44.3 g (0.25 Mol) 2,3,4-Trifluornitrobenzol zugetropft (30 min). Man hält 20 h bei dieser Temperatur, die GC-Analyse zeigt nach dieser Zeit Anteile von 57 % 2,3-Difluor-6-nitro-benzonitril und 43 % 2,3,4-Trifluornitrobenzol, bezogen auf flüchtige Komponenten, in der tiefschwarzen Reaktionsmischung. Man läßt abkühlen, saugt vom festen Rückstand ab und destilliert das Lösungsmittel ab. Es lassen sich im Destillat durch GC-Analyse 15.2 g (86 mMol) 2,3,4-Trifluornitrobenzol quantifizieren, die nicht rückisoliert wurden. Der schwarze Rückstand (31.7 g) wird in 300 ml Dichlormethan gelöst und durch Kieselgel filtriert. Das gesamte Filtrat (gelb) wird am Rotationsverdampfer vom Lösungsmittel befreit und der beigefarbene Rückstand im Vakuum von Restmengen anhaftendem 2,3,4-Trifluornitrobenzol befreit. Man erhält 24.8 g (0.135 Mol, 54 % d. Th., 82 % d. Th. bezogen auf umgesetztes 2,3,4-Trifluornitrobenzol) 2,3-Difluor-6-nitrobenzonitril, das eine für die weitere Umsetzung ausreichende Reinheit (GC > 95 %) aufweist, von dem jedoch zu analytischen Zwecken eine kleine Menge durch präparative Dünnschichtchromatographie gereinigt wird.

Arbeitet man anstatt in tert.-Butanol in 200 g N,N-Dimethylacetamid (DMAc) unter Verwendung von 14.7 g (0.3 Mol) Natriumcyanid bei 40°C, so kann man nach 8 h kein Ausgangsmaterial mehr nachweisen. Nach Filtration vom unlöslichen Rückstand destilliert man etwa 150 g des Lösungsmittels ab und versetzt unter Rühren mit 150 g Wasser. Das ausgefallene Rohprodukt wird bei 0°C abgesaugt und getrocknet (30.7 g, RG (GC) ca. 85 %). Die Substanz kann ohne weitere Reinigung in die nächste Stufe eingesetzt werden.

### 2,3-Difluor-6-nitrobenzonitril:

| Schmp. (DSC): 51.5°C | | | | | |
|---|---|---|---|---|---|
| C₇H₂F₂N₂O₂ (184.102) | ber. | C 45.67 | H 1.09 | F 20.64 | N 15.22 |
| (Masse-%) | gef. | C 45.2 | H 1.3 | F 20.6 | N 15.2 |

IR (KBr, cm⁻¹): 3420, 3090, 2250, 1600, 1545, 1495, 1455, 1350, 1290, 1245, 1140, 1020, 855, 820, 765, 720, 700, 640, 600

¹H-NMR [CDCl₃/ppm]:
- δ =: 7.69 (dd, 1H, Ar-H⁴)
8.24 (ddd, 1H, Ar-H⁵)

¹⁹F-NMR [CDCl₃/ppm]:
- δ =: -122.73 (ddd, 1F, Ar-F³)
-123.78 (ddd, 1F, Ar-F²)

MS: m/z (%) = 46 (8), 61 (14), 68 (11), 75 (24), 87 (20), 88 (99), 99 (10), 112 (26), 126 (38), 138 (42), 154 (11), 167 (5), 184 (100)

### Beispiel 2

24.8 g (0.135 Mol) 2,3-Difluor-6-nitrobenzonitril werden mit 1 g wasserfreiem Calciumchlorid in einem 50 ml-Kolben mit fein ausgezogenem Gaseinleitungsrohr vorgelegt und auf 190°C erhitzt. Bei dieser Temperatur leitet man Chlor mit einem Durchsatz von 2-3 l/h sodaß eine gleichmäßige Entwicklung von nitrosen Gasen auftritt. Nach 12 h Reaktionszeit ist das Ausgangsmaterial weitgehend umgesetzt (< 10 % Restgehalt an Nitroverbindung). Die verbleibenden nitrosen Gase werden mit Luft ausgeblasen und der Rückstand über eine kurze Vigreux-Kolonne im Vakuum abdestilliert. Es werden 15.5 g (89 mMol, 66 %) 2-Chlor-5,6-difluorbenzonitril vom Siedepunkt 70-75°C/3 Torr als leicht gelbliche, ölige Flüssigkeit erhalten (RG (GC) > 95 %).

### 2-Chlor-5,6-difluorbenzonitril:

¹H-NMR [CDCl₃/ppm]:
- δ =: 7.30 (ddd, 1H, J_{BC} = 1.80 Hz, J_{BD} = 3.99 Hz, J_{AB} = 9.01 Hz, Ar-H³ (B)) 7.41 (ddd, 1H, J_{AC} = 7.95 Hz, J_{AD} = 9.05 Hz, J_{AB} = 9.01 Hz, Ar-H⁴ (A))

¹⁹F-NMR [CDCl₃/ppm]:
- δ =: -126.22 (ddd, 1F, J_{BC} = 1.80 Hz, J_{AC} = 7.95 Hz, J_{CD} = 20.14 Hz, Ar-F⁵ (C)) -136.18 (ddd, 1F, J_{BD} = 3.99 Hz, J_{AD} = 9.05 Hz, J_{CD} = 20.14 Hz, Ar-F⁶ (D))

MS: m/z (%) = 61 (6), 68 (5), 75 (6), 87 (9), 88 (22), 93 (4), 99 (4), 112 (6), 118 (4), 137 (6), 138 (19), 146 (3), 148 (1), 173 (100, M⁺), 174 (9), 175 (34), 176 (3)

### Beispiel 3

Von einer Suspension von 5.8 g (0.1 Mol) Kaliumfluorid in 60 g Sulfolan destilliert man zur Trocknung 10 g des Lösungsmittels ab. Man gibt 0.2 g Tetra-n-butylphosphoniumbromid und 15.5 g (89 mMol) 2-Chlor-5,6-difluorbenzonitril zu und erhitzt 8 h auf 190°C. Nach dieser Zeit ist das Ausgangsmaterial vollständig umgesetzt. Man filtriert nach dem Abkühlen vom Salz ab, wäscht mit 20 g warmem Sulfolan nach und destilliert aus dem Filtrat über eine kurze Vigreux-Kolonne 2,3,6-Trifluorbenzonitril ab. Es werden 9.8 g (62 mMol, 70 %) Substanz vom Reingehalt > 90 % erhalten (Verunreinigung im wesentlichen Sulfolan), die ohne weitere Reinigung in die Hydrolyse eingesetzt werden.

### Beispiel 4 (Hydrolysebeispiel)

Zu 20 g 75proz. Schwefelsäure tropft man bei 150°C 9.8 g (63 mMol) 2,3,6-Trifluorbenzonitril in 15 min zu. Nach 3 h läßt sich kein Nitril mehr nachweisen. Die 100° C heiße Lösung wird auf 50 g Eis gegeben und die Mutterlauge mit Methyl-tert.-butylether (MTBE) extrahiert. Durch Trocknen über Magnesiumsulfat und Entfernen des Lösungsmittels erhält man 10.1 g (91 %, 57 mMol) 2,3,6-Trifluorbenzoesäure als leicht gelbliche Kristalle, die zur weiteren Reinigung aus Wasser (15 g) umgelöst werden können (Schmelzpunkt 123°-125°C).

## Patentansprüche

1. 2,3-Difluorbenzonitrile der Formel I 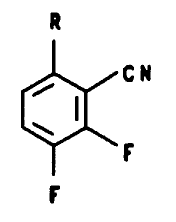 wobei R für NO₂ oder Cl steht

2. Verfahren zur Herstellung von 2,3-Difluorbenzonitrilen der Formel I, dadurch gekennzeichnet, daß man 2,3,4-Trifluornitrobenzol mit einem Cyanid in Gegenwart eines Lösungsmittels zu 2,3-Difluor-6-nitrobenzonitril umsetzt und das 2,3 - Difluor-6-nitrobenzonitril gegebenenfalls mit einem Chlorierungsmittel bei erhöhter Temperatur gegebenenfalls in Anwesenheit eines fluoridfangenden und/oder wasserentziehenden Mittels zu 2-Chlor-5,6-difluorbenzonitril (2,3-Difluor-6-chlorbenzonitril) umsetzt.

3. Verfahren nach Anspruch 2, dadurch gekennzeichnet, daß man als Cyanid ein Alkali- oder Erdalkalimetallcyanid oder ein Cyanid eines Nebengruppenelementes verwendet.

4. Verfahren gemäß den Ansprüchen 2 oder 3, dadurch gekennzeichnet, daß man Natrium, Kalium- oder Kupfercyanid verwendet.

5. Verfahren gemäß einem oder mehreren der Ansprüche 2 bis 4, dadurch gekennzeichnet, daß man das 2,3,4-Trifluornitrobenzol mit Cyanid bei Temperaturen von 20°C bis 120°C bevorzugt zwischen 40°C und 80°C umsetzt.

6. Verfahren nach einem oder mehreren der Ansprüche 2 bis 5, dadurch gekennzeichnet, daß man Cyanide in Anteilen zwischen etwa 1.0 bis 5 Mol, bevorzugt zwischen 1.1 Mol und 3 Mol pro Mol umzusetzendes 2,3,4-Trifluornitrobenzol verwendet.

7. Verfahren nach einem oder mehreren der Ansprüche 2 bis 6, dadurch gekennzeichnet, daß man als Lösungsmittel ein polar aprotisches Lösungsmittel verwendet.

8. Verfahren nach Anspruch 7, dadurch gekennzeichnet, daß man als polar aprotisches Lösungsmittel Aceton, Tetrahydrofuran, Acetonitril, 1,2-Dimethoxyethan, Sulfolan (Tetramethylensulfon), Tetramethylensulfoxid, N,N-Diethylacetamid, N,N-Dimethylacetamid N,N-Dimethylformamid, N-Methylpyrrolidon, Dimethylsulfoxid, Dimethylsulfon, Diphenylsulfoxid, Diphenylsulfon, Tetramethylharnstoff, Tetra-n-butylharnstoff, 1,3-Dimethylimidazolidin-2-on oder Mischungen derselben verwendet.

9. Verfahren nach einem oder mehreren der Ansprüche 2 bis 6, dadurch gekennzeichnet, daß man als Lösungsmittel ein protisches Lösungsmittel verwendet.

10. Verfahren gemäß Anspruch 9, dadurch gekennzeichnet, daß man als protisches Lösungsmittel Wasser oder einen tertiären Alkohol, insbesondere tert.-Butanol verwendet.

11. Verfahren nach einem oder mehreren der Ansprüche 2 bis 10, dadurch gekennzeichnet, daß man das Lösungsmittel in Mengen von 100 Masse-% bis 2000 Masse-%, bezogen auf 2,3,4-Trifluornitrobenzol verwendet.

12. Verfahren nach einem oder mehreren der Ansprüche 2 bis 6, dadurch gekennzeichnet, daß man 2,3,4-Trifluornitrobenzol als Lösungsmittel verwendet.

13. Verfahren nach einem oder mehreren der Ansprüche 2 bis 12, dadurch gekennzeichnet, daß man als Chlorierungsmittel Chlor, Phosphortrichlorid oder Phosphorpentachlorid, insbesondere Chlor verwendet.

14. Verfahren nach einem oder mehreren der Ansprüche 2 bis 13, dadurch gekennzeichnet, daß die Chlorierung bei Temperaturen zwischen 130°C und 250°C, bevorzugt zwischen 160°C und 200°C, besonders bevorzugt zwischen 170°C und 190°C durchgeführt wird.

15. Verfahren nach einem oder mehreren der Ansprüche 2 bis 14, dadurch gekennzeichnet, daß man pro Mol 2,3-Difluor-6-nitrobenzonitril 0,5 bis 20 Mol, insbesondere 0,7 bis 3 Mol Chlorierungsmittel verwendet.

16. Verfahren nach einem oder mehreren der Ansprüche 2 bis 15, dadurch gekennzeichnet, daß man zwischen etwa 10 und etwa 400 ml/gh, insbesondere 20 bis 200 ml/gh Chlor zur Umsetzung des 2,3-Difluor-6-nitrobenzonitrils verwendet.

17. Verfahren nach einem oder mehreren der Ansprüche 2 bis 16, dadurch gekennzeichnet, daß man die denitrierende Chlorierung kontinuierlich oder diskontinuierlich durchführt.

18. Verfahren nach einem oder mehreren der Ansprüche 2 bis 17, dadurch gekennzeichnet, daß als fluoridfangende und/oder wasserentziehende Mittel Calciumchlorid, Calciumhydroxid, Calciumsulfat, Siliciumdioxid, Phosphorpentachlorid oder Phosphorpentoxid, insbesondere Calciumchlorid, Calciumhydroxid, Calciumsulfat oder Siliciumdioxid, verwendet.

19. Verfahren gemäß den Ansprüchen 2 bis 18 dadurch gekennzeichnet, daß man in allen Verfahrensstufen bei Unter-, Über- oder Atmosphärendruck arbeiten kann.

20. Verwendung von 2-Chlor-5,6-difluor-benzonitril, dadurch gekennzeichnet, daß man die Verbindung mit Fluoriden zu 2,3,6-Trifluorbenzonitril und anschließend durch Hydrolyse zu 2,3,6-Trifluorbenzoesäure umsetzt.

## Claims

1. A 2,3-difluorobenzonitrile of the formula I 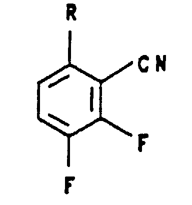 in which R is NO₂ or Cl.

2. A process for the preparation of 2,3-difluorobenzonitriles of the formula I, which comprises reacting 2,3,4-trifluoronitrobenzene with a cyanide in the presence of a solvent to give 2,3-difluoro-6-nitrobenzonitrile and optionally reacting the 2,3-difluoro-6-nitrobenzonitrile with a chlorinating agent at elevated temperature, optionally in the presence of a fluoride-trapping and/or dehydrating agent, to give 2-chloro-5,6-difluorobenzonitrile (2,3-difluoro-6-chlorobenzonitrile).

3. The process as claimed in claim 2, wherein the cyanide used is an alkali metal or alkaline earth metal cyanide or a cyanide of a subgroup element.

4. The process as claimed in claim 2 or 3, wherein sodium, potassium or copper cyanide is used.

5. The process as claimed in one of more of claims 2 to 4, wherein the 2,3,4-trifluoronitrobenzene is reacted with cyanide at temperatures from 20°C to 120°C, preferably between 40°C and 80°C.

6. The process as claimed in one or more of claims 2 to 5, wherein cyanides are used in proportions between about 1.0 and 5 mol, preferably between 1.1 mol and 3 mol, per mole of 2,3,4-trifluoronitrobenzene to be converted.

7. The process as claimed in one or more of claims 2 to 6, wherein the solvent used is a polar aprotic solvent.

8. The process as claimed in claim 7, wherein the polar aprotic solvent used is acetone, tetrahydrofuran, acetonitrile, 1,2-dimethoxyethane, sulfolane (tetramethylene sulfone), tetramethylene sulfoxide, N,N-diethylacetamide, N,N-dimethylacetamide, N,N-dimethylformamide, N-methylpyrrolidone, dimethyl sulfoxide, dimethyl sulfone, diphenyl sulfoxide, diphenyl sulfone, tetramethylurea, tetra-n-butylurea, 1,3-dimethylimidazolidin-2-one or mixtures thereof.

9. The process as claimed in one or more of claims 2 to 6, wherein the solvent used is a protic solvent.

10. The process as claimed in claim 9, wherein the protic solvent used is water or a tertiary alcohol, especially tert-butanol.

11. The process as claimed in one or more of claims 2 to 10, wherein the solvent is used in amounts of 100% by weight to 2000% by weight, based on 2,3,4-trifluoronitrobenzene.

12. The process as claimed in one or more of claims 2 to 6, wherein 2,3,4-trifluoronitrobenzene is used as the solvent.

13. The process as claimed in one or more of claims 2 to 12, wherein the chlorinating agent used is chlorine, phosphorus trichloride or phosphorus pentachloride, especially chlorine.

14. The process as claimed in one or more of claims 2 to 13, wherein the chlorination is carried out at temperatures between 130°C and 250°C, preferably between 160°C and 200°C, particularly preferably between 170°C and 190°C.

15. The process as claimed in one or more of claims 2 to 14, wherein 0.5 to 20 mol, especially 0.7 to 3 mol, of chlorinating agent are used per mole of 2,3-difluoro-6-nitrobenzonitrile.

16. The process as claimed in one or more of claims 2 to 15, wherein between about 10 and about 400 ml/gh, especially 20 to 200 ml/gh, of chlorine are used to convert the 2,3-difluoro-6-nitrobenzonitrile.

17. The process as claimed in one or more of claims 2 to 16, wherein the denitrating chlorination is carried out continuously or batchwise.

18. The process as claimed in one or more of claims 2 to 17, wherein the fluoride-trapping and/or dehydrating agents used are calcium chloride, calcium hydroxide, calcium sulfate, silicon dioxide, phosphorus pentachloride or phosphorus pentoxide, especially calcium chloride, calcium hydroxide, calcium sulfate or silicon dioxide.

19. The process as claimed in claims 2 to 18, wherein all the process steps can be carried out under reduced pressure, excess pressure or atmospheric pressure.

20. Use of 2-chloro-5,6-difluorobenzonitrile, which comprises reacting the compound with fluorides to give 2,3,6-trifluorobenzonitrile and then hydrolyzing the latter to give 2,3,6-trifluorobenzoic acid.

## Revendications

1. 2,3-difluorobenzonitriles de formule I 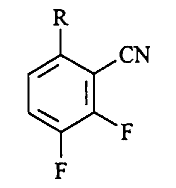 dans laquelle R est NO₂ ou Cl.

2. Procédé de préparation de 2,3-difluorobenzonitriles de formule I, caractérisé en ce que l'on fait réagir du 2,3,4-trifluoronitrobenzène avec un cyanure en présence d'un solvant pour former le 2,3-difluoro-6-nitrobenzonitrile, et on fait éventuellement réagir le 2,3-difluoro-6-nitrobenzonitrile avec un agent de chloration à température élevée, éventuellement en présence d'un agent fixateur de fluorures et/ou d'un agent déshydratant, pour former le 2-chloro-5,6-difluorobenzonitrile (2,3-difluoro-6-chlorobenzonitrile).

3. Procédé selon la revendication 2, caractérisé en ce que l'on utilise comme cyanure un cyanure de métal alcalin ou de métal alcalino-terreux ou un cyanure d'un élément d'un sous-groupe.

4. Procédé selon les revendications 2 ou 3, caractérisé en ce que l'on utilise du cyanure de sodium, de potassium ou de cuivre.

5. Procédé selon l'une ou plusieurs des revendications 2 à 4, caractérisé en ce que l'on fait réagir le 2,3,4-trifluoronitrobenzène avec un cyanure à des températures de 20°C à 120°C, de préférence de 40°C à 80°C.

6. Procédé selon l'une ou plusieurs des revendications 2 à 5, caractérisé en ce que l'on utilise les cyanures en des quantités comprises entre environ 1,0 et 5 mol, de préférence entre environ 1,1 mol et 3 mol par mol de 2,3,4-trifluoronitrobenzène à faire réagir.

7. Procédé selon l'une ou plusieurs des revendications 2 à 6, caractérisé en ce que l'on utilise comme solvant un solvant polaire aprotique.

8. Procédé selon la revendication 7, caractérisé en ce que l'on utilise comme solvant polaire aprotique l'acétone, le tétrahydrofurane, l'acétonitrile, le 1,2-diméthoxyéthane, le sulfolane (tétraméthylènesulfone), le tétraméthylènesulfoxyde, le N,N-diéthylacétamide, le N,N-diméthylacétamide, le N,N-diméthylformamide, la N-méthylpyrrolidone, le diméthylsulfoxyde, la diméthylsulfone, le diphénylsulfoxyde, la diphénylsulfone, la tétraméthylurée, la tétra-n-butylurée, la 1,3-diméthylimidazolidine-2-one ou des mélanges de ces solvants.

9. Procédé selon l'une ou plusieurs des revendications 2 à 6, caractérisé en ce que l'on utilise comme solvant un solvant protique.

10. Procédé selon la revendication 9, caractérisé en ce que l'on utilise comme solvant protique de l'eau ou un alcool tertiaire, en particulier le tert-butanol.

11. Procédé selon l'une ou plusieurs des revendications 2 à 10, caractérisé en ce que l'on utilise le solvant en des quantités de 100 % en masse à 2000 % en masse par rapport au 2,3,4-trifluoronitrobenzène.

12. Procédé selon l'une ou plusieurs des revendications 2 à 6, caractérisé en ce que l'on utilise le 2,3,4-trifluoronitrobenzène comme solvant.

13. Procédé selon l'une ou plusieurs des revendications 2 à 12, caractérisé en ce que l'on utilise comme agent de chloration du chlore, du trichlorure de phosphore ou du pentachlorure de phosphore, en particulier du chlore.

14. Procédé selon l'une ou plusieurs des revendications 2 à 13, caractérisé en ce que la chloration s'effectue à des températures comprises entre 130°C et 250°C, de préférence entre 160°C et 200°C, de façon particulièrement préférée entre 170°C et 190°C.

15. Procédé selon l'une ou plusieurs des revendications 2 à 14, caractérisé en ce que l'on utilise 0,5 à 20 mol, en particulier 0,7 à 3 mol d'agent de chloration par mol de 2,3-difluoro-6-nitrobenzonitrile.

16. Procédé selon l'une ou plusieurs des revendications 2 à 15, caractérisé en ce que l'on utilise entre environ 10 et environ 400 ml/g.h, en particulier de 20 à 200 ml/g.h de chlore pour la transformation du 2,3-difluoro-6-nitrobenzonitrile.

17. Procédé selon l'une ou plusieurs des revendications 2 à 16, caractérisé en ce que l'on effectue la chloration avec dénitration de façon continue ou discontinue.

18. Procédé selon l'une ou plusieurs des revendications 2 à 17, caractérisé en ce que l'on utilise comme agent fixateur de fluorures et/ou comme agent déshydratant le chlorure de calcium, l'hydroxyde de calcium, le sulfate de calcium, le dioxyde de silicium, le pentachlorure de phosphore ou le pentoxyde de phosphore, en particulier le chlorure de calcium, l'hydroxyde de calcium, le sulfate de calcium ou le dioxyde de silicium.

19. Procédé selon l'une ou plusieurs des revendications 2 à 18, caractérisé en ce que l'on peut travailler dans toutes les étapes du procédé sous pression réduite, sous une surpression ou sous la pression atmosphérique.

20. Utilisation du 2-chloro-5,6-difluorobenzonitrile, caractérisée en ce que l'on fait réagir le composé avec des fluorures pour former le 2,3,6-trifluorobenzonitrile, puis par hydrolyse pour former l'acide 2,3,6-trifluorobenzoïque.
